# EUROPEAN PATENT SPECIFICATION

(11) **EP 2 468 319 B1**
(45) Date of publication and mention of the grant of the patent: **23.11.2016**
(21) Application number: 10460045.7
(22) Date of filing: 29.11.2010
(51) Int. Cl.: A61L 29/08, A61L 29/16

(54) **New nanoproduct useful in prophilaxis, diagnostics and medical and veterinary treatment**
Neues Nanoprodukt zur Verwendung bei der Prophylaxe, Diagnose sowie bei der medizinischen and veterinärmedizinischen Behandlung
Nouveau nano-produit utile dans la prophylaxie, diagnostic et traitement médical et vétérinaire

(43) Date of publication of application: 27.06.2012
(73) Proprietor: Eurochit Danuta Kruszewska, 02-765 Warszawa (PL)
(72) Inventor: Kruszewska, Danuta, 02-765 Warszawa (PL)
(74) Representative: Malewska, Ewa

(56) References cited:
- EP-A1- 1 917 959
- WO-A2-2005/021575
- US-A1- 2003 018 306
- DATABASE CAPLUS [Online] CHEMICAL ABSTRACTS SERVICE, COLUMBUS, OHIO, US; 2008, Xu, Jianwen; Sun, Huabai: "Tracheal catheter with improved antibacterial and lubricant effects", XP002638794, Database accession no. 2008:302494 & CN 101 134 125 A (JIANWEN XU [CN]) 5 March 2008 (2008-03-05)
- DATABASE WPI Week 201034 Thomson Scientific, London, GB; AN 2010-F24852 XP002638795, LI J; LI J: "Tracheal catheter, has antibacterial layer coated on external surfaces of airbag and front end tube, where external surface of antibacterial layer is coated with lubricant layer", & CN 201 445 692 U (LI Z) 5 May 2010 (2010-05-05)
- HSIEH M ET AL: "Probiotic lactobacillus reuteri RC-14 and lactobacillus rhamnosus GR-1 for prevention of urinary tract infections in catheterization-dependent girls with spina bifida", JOURNAL OF PEDIATRIC UROLOGY, ELSEVIER, NL, vol. 5, 1 April 2009 (2009-04-01), page 47, XP026050651, ISSN: 1477-5131, DOI: DOI:10.1016/J.JPUROL.2009.02.067 [retrieved on 2009-04-17]
- REID GREGOR; TIESZER CHRISTINA: "USE OF LACTOBACILLI TO REDUCE THE ADHESION OF STAPHYLOCOCCUS AUREUS TO CATHETERS.", INTERNATIONAL BIODETERIORATION AND BIODEGRADATION, vol. 34, no. 1, 1994, pages 73-83, XP002641996,

## Description

The invention relates to a catheter for insertion into body vessels, ducts and/or cavities, improved due to the use of a new nanoproduct useful in the prophylaxis, diagnostics and medicine, both human and veterinary. In particular, the invention relates to a urethral catheter.

Catheters are widely used in human and veterinary medicine, as to enable drainage or administration of fluids or gases, and for insertion of endoscope, tubes and surgical instruments.

However, apart from undoubted benefits, undesirable effects are also associated with the use of catheters.

Despite the efforts towards maintaining the conditions of sterility and hygiene, the use of a catheter increases the risk of the entry of pathogenic agents from the external environment, as well as dissemination of the organism's own natural microbiota colonizing specific sites/niches in the organism, becoming pathogenic microbiota at the site of catheter placement. The occurrence of pathogens in the region of catheterization causes the development of infection (of various intensity).

In addition, the insertion of a catheter into a body vessel, duct or cavity is a procedure both painful and stressful for a patient. Moreover, as a result of a long-term contact of tissues with the catheter and agents flowing through the catheter there may develop inflammations, and frequently, also allergic reactions.

According to the intended use, catheters are produced of various plastics. Known catheters manufactured of medical polyvinyl chloride (PVC) - mainly of the Nelaton type, are used for a short-term catheterization and kept in a patient's body for a period up to 3 days. Such catheters constitute over 80% of the catheters used. Other polymers employed for production of catheters are polyurethanes (PU), silicon or natural latex and their derivatives. Mainly Folley catheters (with a fixation device) are produced of silicon, which Folley catheters are medical products for long-term use and may be kept in place for up to 3 months.

Unfortunately, high quality polyurethane and silicon catheters are considerably more expensive than those produced of PCV, and use of natural latex is while at present not recommended as it may cause allergic reactions.

Common problems connected with the necessity to use catheters in human and veterinary medicine are well illustrated by the phenomena accompanying the catheterization of patients with urological problems. Despite the achievements in medicine and health care, catheter-associated urinary tract infections (CAUTI) still remain among the most frequently occurring hospital infections. For example, in the USA each year, more than 1 million hospitalized patients need to be treated due to CAUTI.

A permanent indwelling urethral catheter is one of the causes of the occurrence of hospitals-acquired urinary tract infections (UTI). In many cases, hospital infections are caused by multidrug-resistant bacterial strains and require complex and expensive treatment with antibiotics.

Each year in the Unites States, catheters are inserted in more than 5 million patients in intensive-care units, and even more in nursing homes. In Poland, 200,000 urethral catheters are used annually. It is estimated that the risk of infection associated with short-term catheter use is 5-6% per one day of catheterization. Infections related with long-term catheterization lasting for more than 7 days are usually caused by a number of bacterial strains which form a biofilm on the catheter surface, and may lead to catheter clogging.

Biofilm is generated by various microbes, including ureolytic bacteria, non-pathogenic commensals, permanently and naturally colonizing the surface of the epithelium, pathogens, including microorganisms causing urogenital system infections (e.g. *Proteus mirabilis*). The common feature of ureolytic bacteria is their ability to make use of urea present in their environment (tissues) - mainly as a source of nitrogen necessary for survival, which use involves urease. Urease (including bacterial urease), hydrolyses urea to ammonia and carbon dioxide. Examples of nitrogen-assimilating bacteria by means of their own ureases are biofilm forming bacteria.

Ureolytic bacteria, even though they are not the main etiological factor in urinary tract infections in healthy organisms, are often associated with infections in patients with urinary tract disorders. Ureolytic bacteria are responsible for the forming of biofilm and mineralization of deposits on catheters and other medical instruments. Among the consequences of urinary tract infections caused by urease-producing microorganisms is nephrolithiasis, accompanied by supersaturation of urine with mineral salts: ammonium-magnesium phosphate (struvite), calcium phosphate, oxalates and urates. In physiological conditions, urea does not contain these salts in amounts indicating the formation of sand or stones.

The infected kidney stones formation is associated with the urinary tract infections caused by microorganisms of the following genera: *Proteus, Ureaplasma, Klebsiella*, *Pseudomonas, Staphylococcus, Providencia*, and *Corynebacterium.*

Another undesirable effects of these infections are pathological processes within the kidney parenchyma. Bacteremia is one of serious complications which may occur as a result of cathetrization.

In general, morbidity due to urinary tract infections depends on the characteristics and status of a patient, the pathogenic microorganism and hospital environment. Usually, little can be done to reduce factors associated with the host (organism), because most of them are intrinsic either to a patient (host) or to bacteria.

Age, self-catheterization, and total or continuous incontinence by patients with neurogenic disorders are a few of the factors of hospital-acquired urinary tract infections. Such patients suffer as a result of infection associated with catheterization. In hospital conditions, important risk factors cover the type of catheter, duration of insertion, type of placement, and use of antibacterial or antiseptic substances. Microbes permanently colonizing the urinary tract represent the major source of microorganisms causing catheterization-related infections. A significant number of bacteria are found in urine samples, among them *E coli* strains prevail. The infection usually takes an asymptomatic course, while in 20% of cases it is symptomatic, irrespective of antibiotic treatment. The index of bleeding/blood traces ratio (connected with catheter insertion) is also high and occurs in one per five cases. Seventy five percent of patients catheterized for a period longer than one year develop UTI symptoms of varying intensity.

Patients of both sexes, especially the elderly undergoing long-lasting treatment with intermittent catheterization, usually complain also of physical and psychological complications related to the treatment.

Urethral catheters are usually manufactured from natural latex or synthetic polymers. Contemporary catheters available on the market differ with respect to shape, method of dilatation, and materials of which they have been manufactured. These characteristics cause differences in the protocols of use of individual catheters.

In the prior art, attempts have been undertaken to enhance the usability values of catheters to be applied in human and veterinary medicine by coating them with chemotherapeutics and impregnation with antiseptics or other agents (e.g. anticoagulants). The first remedy is the application of hydrogel on the catheter surface directly before catheterization in order to decrease its friction coefficient and reduce pain. It is mainly composed of polyvinyl pyrrolidone (PVP), usually in combination with iodide acting antiseptically. However, it requires additional operations and the gel applied is easily rinsed away, therefore the antiseptic action is limited in time. More advanced coatings are permanently attached to the catheter surface and allow decrease in the friction coefficient, not only during insertion, but also during catheter removal. A permanent hydro gel coating may also serve as an antimicrobial drug reservoir that slows down surface colonization. Nevertheless, the problem of infections associated with long lasting catheterization has not been fully resolved. In the case of long term placement of a catheter within the organism, the process of antibacterial agent release should be controlled and slow to maintain its bactericidal properties for the whole period of catheterization, which has not been accomplished in a sufficient degree.

There are many known methods of application of antibacterial coatings on natural or polymer tubes. Coating techniques vary as well. The hydrogel coating technique is advantageous due to high biocompatibility of the coating, low friction, decreased bacteria adhesion, and a possibility of incorporation of drug into the coating.

In EP 1917959 the use of alpha-ketoglutarate has been disclosed for production of an agent against the deposit and infectious stones formation in the urogenital system by ureolytic bacteria.

In WO 2005/021575 a composition and methods of employing said composition for treating or preventing microbial associated infections and diseases have been disclosed. More particularly, the disclosure covers bacterial proteins, peptides and amino acids which are by-products of bacteria, in particular *Lactobacillus* and more specifically *Lactabacillus* strains GR-1 and RC-14, in compositions that can treat and prevent microbial-associated infections and diseases, by altering, for example, down-regulating, virulence properties of pathogenic organisms. In one of its aspects, the proposed solution is directed to the infections caused by biofilms and a method for reducing the risk of infections associated with medical devices such as catheteres, stants, valves, implants and the like, comprising delivery of at least one signal molecule produced by non-pathogenic microorganisms, such as various *Lactobacillus* strains including *L*. *reuteri*, to said medical device or to the sites surrounding said medical device.

Despite the studies conducted and efforts undertaken to solve the above-mentioned problems, the whole clinical world awaits a solution which would enable furnishing patients with catheters, especially urethral catheters, for a long periods of time.

The primary aim of the present invention is the provision of specialist catheters which would be long-lasting, functional and simultaneously, appropriate for long-term keeping inside a patient's body and could reduce the risk of infection.

Another objective of the invention is the use of nanotechnology to provide natural antibacterial catheters, including urethral catheters.

A further aim of the invention is the use of nanotechnology to provide natural antimicrobials, which would provide the certainty of endogenous biodegradation, for body fluids and tissues, acting from the surface of catheters.

At present, it has been unexpectedly found out that the above-mentioned and other goals may be achieved by a solution according to the invention, based on coating the surface of a catheter with nanocoatings of various substances, primarily with the use in the composition or at least one nanocoating of a component originating from a new strain of lactic acid bacteria, reducing the risk of viral and bacterial infections.

Surprisingly, it was also observed that incorporation of specific vitamins in nano-form and alpha-ketoglutarate into nanocoatings of a catheter according to the invention provides a synergistic effect with respect to the prevention of infections and inflammations of the tissues remaining in a long-term contact with the catheter.

Catheter for insertion into body vessels, ducts and/or cavities, for use in prophylaxis, diagnostics and medicine, both human and veterinary, made of plastic and coated with a protective lubricant layer, in accordance with the invention has an outer nanocoating of biocompatible polymer capable of forming gel with water, permanently attached to said plastic either directly or through a nanocoating of polymer chemically bonded to the catheter material and having antibacterial properties, wherein at least one of the nanocoating comprises thermally inactivated cultures of *Lactobacillus reuteri* DAN080 strain with deposit number DSM 15693, said cultures having antimicrobial and anti-inflammatory activity, as well as an optional addition of vitamin D and E in form of nanoparticles.

According to the invention the biocompatible polymer is polyvinyl pyrrolidone and thickness of the nanocoating made of this polymer is about 50,000 C-C bonds (10 nm).

Preferably, the polymer having antibacterial properties is a salt of chitosan and small organic acid, preferably alpha-ketoglutaric acid.

According to the invention, in nanocoating of biocompatible polymer and/or in nanocoating of polymer having antibacterial properties there are dispersed additional active agents selected from the group comprising chitosan alpha-ketoglutarate, chitosan citrate, chitosan lactate with antimicrobial and anti-inflammatory activity, small di-carboxylic acid, silver nanoparticles, vitmain D and E in the form of nanopowder coated with a protective coating and combinations thereof.

The invention also covers a kit for catheterization, comprising a catheter and a vial with water for injection (sterile) and antimicrobial and anti-inflammatory agent to be administered orally in the form of thermally inactivated cultures of *Lactobacillus reuteri* DAN080 strain with deposit number DSM 15693 at a dose of 10⁶ cells, for daily administration for the period of catheterization, preferably for oral administration 8 hours prior to catheterization, or for administration into the body cavity 15 minutes before catheterization.

In the kit according to the invention, the water container is preferably fixed at the catheter tip, and the container has a partition separating water from the catheter, the partition being destroyed by rotation of the container against the catheter while protruding the catheter from the packaging.

The catheter according to the invention satisfied all the above-mentioned requirements and is user-friendly, acceptable for the catheterized patients, the medical staff and medical opponents. According to the invention nanocoatings were developed for on catheters made of PVC and of silicon. The hydrogel nanolayer is permanently attached to the polymer surface, and not only decreases the friction coefficient, thus reducing pain experienced by the patient catheterized, but also contains an additive being an agent which in contact with the patient's body tissues reduces patient's stress associated with catheter insertion and removal. Simultaneously, the same additive induces an increase of lysozyme level in tissues remaining in contact with the catheter, thus decreasing the risk of viral, bacterial infection, due to a very wide spectrum of lysozyme activity. The presence of a new coating on the catheter surface reduces the formation of biofilm on the catheter surface. This nanocoatings contains also other active substances released slowly in a controlled manner.

Surface nanoengineering applied in accordance with the present invention allows providing a coating which releases a medicament on demand. One of the signals triggering release of the drug may be, for example, a change of pH of the environment caused by bacterial growth. This targeted drug release is much more effective and shows less side effects. In addition, by the proper selection of the composition of coating layers dedicated catheters are obtained, having varied destination, adjusted to the patient's status. The use of a coating made of PVP/chitosan salt with a chemically bonded drug (e.g. small dicarboxylic acid) ensures achievement of the goals assumed.

The present catheter ensures the expected progress in medical care due to the possibility of making use of nanotechnology for delivery of natural antimicrobial agents, acting on the surface of the catheter according to the invention. The catheter being coated with nanocoating is more convenient in use and safer for a patient. The coating reduces pain connected with insertion of the catheter and significantly decreases the probability of infection. Moreover, all the active substances used do not induce any undesirable side effects in patient.

Due to the present invention based on the use of nanotechnology, the role of protective coatings on catheters is maximized thanks to enhancement of antimicrobial properties of nanocoatings and targeted release on demand of an active substance. It is most important that the catheters according to the invention will save pain for catheterized patients and decrease the number of infections, while in view of character of the compound employed eliminate the development of drug-resistance in microorganisms induced by classic antibiotics.

The invention is described below in more detail, with the reference to the drawing in which:
Fig. 1 presents the relationship between activity of lysozyme (U/L) in rat blood and intragastrical administration of: DAN080 - 10⁶ cells *Lactobacillus reuteri* DAN080; DAN080P - thermally killed 10⁶ cells *Lactobacillus reuteri* DAN080; ChAKG - chitosan alpha-ketoglutarate; SF - saline;
Fig. 2a-2f illustrate the results of open field tests performed on rats treated with *L. reuteri* DAN080, inactivated *L. reuteri* DAN080, chitosan alpha-ketoglutarate, and of open field behavioral tests performed on rats on rats treated with *L. reuteri* DAN080, inactivated *L. reuteri* DAN080, and chitosan alpha-ketoglutarate and saline.
Fig. 3 presents the electrophoretic separation of supernatants of *L. reuteri* DAN080 cultures. The growth time was from 1 - 10 hours.

New coatings were investigated, developed as external layers for urethral catheters of PVC, polyurethane and silicon. The catheters are impregnated with the known nanogel (polyvinyl pyrrolidone - PVP), with chemically bonded chitosan salts exhibiting antimicrobial activity (citric or lactic acid salts or alpha-ketoglutarate, or mixtures thereof in various proportions and amounts).

Chitosan salts of antimicrobial activity (citric or lactic or alpha-ketoglutaric acid salts, or mixtures thereof in various proportions and amounts) may also form a coating onto which the known nanogel is applied in a non-modified form or modified with above-mentioned chitosan salts of antimicrobial activity.

The subsequent layer may be formed of vitamin D and chitosan salts of antimicrobial activity (citric or lactic or alpha-ketoglutaric acid salts, or mixtures thereof in various proportions and amounts) in alternate sequence, with a closing layer of hydrogel in non-modified form or modified with chitosan salts of antimicrobial activity (citric or lactic or alpha-ketoglutaric acid salts, or mixtures thereof in various proportions and amounts).

Also possible is separate stratification of the catheter with extracellular metabolites of *L. reuteri* DAN080 and with silver or modification therewith the nanogel which is coating the catheter on a side facing the site of catheter activity.

The effectiveness of the new coatings was evaluated with regard to reduction of infections and reduction of allergic reactions. The relationship between the intensity of colonization of the catheter surface by microorganisms and the degree of surface incrustation was analyzed.

### Detailed description of invention

The terms used in the presented description should be understood in their common basic meaning, unless defined otherwise below.

Nanocoating means known nanogel (polyvinyl pyrrolidone - PVP or other), in which polymers used are chemically bonded to chitosan salts of antimicrobial activity (citric or lactic or alpha-ketoglutaric acid salts, or mixtures thereof in various proportions and amounts).

Chitosan salts of antimicrobial activity (citric or lactic or alpha-ketoglutaric acid salts, or mixtures thereof in various proportions and amounts) may also form a coating which may be coated with a known nanogel in a non-modified form or modified with the above-mentioned chitosan salts of antimicrobial activity.

The subsequent coating may form vitamin D alternatively with chitosan salts of antimicrobial activity (citric or lactic or alpha-ketoglutaric acid salts, or mixtures thereof in various proportions and amounts), and a final coating of hydrogel in non-modified form or modified with chitosan salts of antimicrobial activity (citric or lactic or alpha-ketoglutaric acid salts, or mixtures thereof in various proportions and amounts).

Also possible is separate stratification of the catheter with extracellular metabolites of *L. reuteri* DAN080 as deposited under the number: DSM 15693 and with silver or modification therewith the nanogel which is coating the catheter on a side facing the site of catheter activity.

Two types of polymers were used as a basic material for manufacturing the catheters: PVC and silicon.

PVC is a cheap polymer, the safety of which has been confirmed for many years. At present, a new generation of plasticizers is used, additionally increasing safety of PVC. Silicon is an expensive polymer; however, it is characterized by a very high compatibility.

According to the invention a catheter made of PVC or silicon is coated with a polymer nanocoating made of polyvinyl pyrrolidone (PVP). Only in contact with water PVP forms a thick jelly solution which lubricates the polymer surface.

Polymer coating made of PVP may also be applied on catheters made of polyurethane or natural latex.

PVP is widely used in pharmacology for the production of biomaterials intended for contact with blood, in results of its biocompatibility (lack of toxic effect, including degradation effect on blood cells - hemolysis, lack of effect on the host immune system). An advantage of nanocoating made of PVP is that this coating is resistant to activity of microorganisms, including pathogenic organisms.

The only drawback of such coating is the need for wetting the catheter prior to insertion the procedure; however, this problem can be easily solved in accordance with the invention inside the package, when using a kit for catheterization according to the invention.

According to the invention, nanocoating made of PVP is chemically bonded to a polymer of antimicrobial activity, such as chitosan salts. This is a polymer obtained from *Crustacea* shells. Chitosan salts are known for their use in medicine. They are safe, bioavailable and biodegradable.

In order to prove an unexpected synergism, the coatings made of chitosan salts and PVP were examined separately and in combination. In addition, the composition of individual coatings was enriched with other active substances increasing the spectrum of activity of the surface of the catheter according to the invention, such as extracellular metabolites of *L*. *reuteri* DAN080 (deposit DSMZ - access number - DSM 15693 - in accordance with a Budapest Treaty on the International Recognition of the Deposit of Microorganisms for the Purposes of Patent Procedure, on 20 June 2003), and vitamin D in the form of nanoparticles and/or nanoparticles of silver.

Forming of solid polymer coating may be proven by two methods - physical and chemical, based on covalent polymer chains bonded through covalent bonds. Physical anchoring of polymer chains can be achieved by forming a nanocoating of discontinuous PU layer and application of PVP solution.

Due to London forces, the PVP chain is partially immersed in the basic polymer and partially protrudes therefrom. This nanocoating, having thickness of about 50,000 C-C bonds (10 nm), when immersed in water forms a sort of brush with excellent lubricative properties. This original technology has already been developed.

Alternatively, on the surface of the basic polymer, a desired layer is deposited by the method of forming free radicals by hydrogel absorption. Such free radicals are very active and easily 'catch' other chemical substances forming stable covalent bonds.

The application of both technologies is possible, as both of them provide the desired coatings.

Nanocoatings obtained on the polymer surface are tested to evaluate their biocompatibility, development of a biofilm and colonization by microorganisms. Their friction coefficient against pig tissue is also examined. The evaluation is performed making use of a specially constructed device, improved to meet the needs of the current invention. The optimum friction coefficient ensures painless insertion of the catheter, however, without any risk of its slipping out.

The novel properties of the external nanocoatings of the catheter according to the invention were achieved by physical and/or chemical bonding of active agents.

An active agent of the external catheter nanooatings is the component originating from the new DAN080 strain of lactic acid bacteria *L. reuteri* identified by the present inventor, deposited on June 20, 2003 - in accordance with a Budapest Treaty on the International Recognition of the Deposit of Microorganisms for the Purposes of Patent Procedure, in DSMZ collection - Deutsche Sammlung von Mikroorganismen und Zellkulturen GmbH, in Braunschweig, DE, access number: DSM 15693.

It has been now unexpectedly found out that thermally inactivated cultures of *L*. *reuteri* DAN080 are a desired component of the external nanocoating, or one of the layers which coat the catheter according to the invention.

After the specified period of growth, the *L. reuteri* DAN080 culture is centrifuged, and the liquid, concentrated supernatant and dried supernatant is the product of specific capabilities and activity with respect to the regulation of bacteria growth *in vitro* and *in vivo.* After the electophoretic separation performed using a liquid supernatant, concentrated supernatant and dried supernatant collected at a specific time, specific proteins were visualized, the proteins having molecular weight within the range of 150 - 22 kD and smaller, which are responsible for homeostasis and bacteria growth regulation in the patient's body. These proteins, both in an isolated and purified form, and also in the form of a liquid supernatant, concentrated supernatant and dried supernatant, collected after the proper period of culturing the *L. reuteri* DAN080 culture, independently or in a mixture with other lactic acid bacteria isolated by and being the property of the present inventor - Danuta Kruszewska, confer new, unexpected properties to nanocoatings coated on the catheter according to the invention, which properties increase the safety and comfort for patients, while showing homeostatic and regulatory effect on bacterial growth on the surface of the catheter according to the invention.

Extracellular metabolites of the *L. reuteri* DAN080 are used in combination with other known agents of antibacterial activity, according to the present disclosure. The methods of isolation, culturing the *L. reuteri* DAN080 bacteria and collection, isolation and purification of extracellular metabolites of the *L. reuteri* DAN080 are disclosed in a parallel patent application claiming the same priority date as the present invention.

Apart from thermally inactivated cultures of the *L. reuteri* DAN080, vitamins are also used as active agents, especially vitamins D and E in the form of nanopowders, in order to enhance the patient's immune mechanisms, and also as agents reducing the growth of microorganisms and the formation of biofilm according to the present invention nanoparticles of silver, small dicarboxylic acid and chitosan salts may be used. In order to gradually release the above-mentioned active agents, known compositions for delayed release were used, for example compositions soluble in physiological fluids for coating the hydrogel. The selection of the used technical means for delayed release of the substances depends on the necessity of preserving their properties for the time adjusted to the anticipated period of catheter placement inside patient's body, considering the diffusion coefficient and/or degree of bonding on the surface.

### Description of experiments

### Preparation of chitosan salts

The raw material used for producing proper chitosan salts is technical chitin obtained from *Basinomycetes* (*Lentinus edodes,* Le 323), according to the publication No. PL 384836 - "Method for obtaining fungal chitosan' of 12 October 2009, or from the scutum of Antarctic krill (*Euphausia superba*). The residues of organic and inorganic contaminants are removed from the polymer in the processes of demineralization and deproteinization. A part of the chitin thus obtained is subjected to the process of chemical decomposition in order to reduce its polymerization degree to the desired level. This allows obtaining chitosans with similar deacetylation levels and different molecular weights. Chitosan is obtained in the process of alkaline deacetylation. Its properties are modified by changing the reaction time and temperature. Salts are obtained in the reaction of chitosan with organic acid(s) in aqueous environment. Thus obtained salt solution is lyophilized. The evaluation of the properties of raw material and products is monitored.

In order to obtain (in laboratory conditions) chitosans of various molecular weights and deacetylation levels the following procedure is applied:
preparation of the raw material for the production of chitosan: technical grade chitin is purified, the molecular weight of the polymer is modified in order to obtain chitin having various polymerization levels. Chitosan of the required properties is obtained through control of parameters of the deacetylation process.

Chitosan salts obtained in laboratory conditions are tested for their antimicrobial properties.

The biological testing of chitosan salts that facilitates quick assessment of their antimicrobial activity, allows monitoring, control and selection of the optimum parameters of their production process, especially the ranges within which the deacetylation level and molecular weight of chitosans should be modified.

The optimization of the method for obtaining salts is carried out from the aspect of the most intensive biological activity after the sterilization process, taking into consideration the destination of the catheter and the environment in which it will be used.

Adhesion of multi-drug-resistant bacterial strains to coated and non-coated surfaces of the polymers tested.

The pathogenesis of many bacteria is associated mainly with the ability of these organisms to irreversibly adhere to polymer surface and to produce an extracellular glycocalyx in the course of colonization.

The percentage of adherence is defined as a rate of CFU recovered from the polymer tested to CFU of marker bacteria (multi-drug-resistant strains) in the culture fluid.

Antimicrobial activity of chitosan salts with relation to multi-drug-resistant microorganisms

Each sample is cultured together with one of the tested strains (CFU 10³). At various time points (0, 30, 60 and 120 min) after incubation at the temperature of 37°C samples are collected, vortexed and placed on plates plated with agar solidified medium. The CFU values are counted after incubation of microorganisms at the temperature of 37°C for 48 hours. The CFU counts in samples taken before incubation is used to calculate the reduction of CFU.

Studies on animals were based on the non-infectious animal model of the adult rats. Six-month-old Sprawgue-Dawley females rats were used with the weight of ± 350 g (n=90). The rats were divided into 9 groups. Into the urinary tract of rats (n=36; 3 groups) polymer rods were inserted covered with antibacterial and lubricous coatings. Impregnated rods were made of PVC, polyurethane and silicon, respectively. Another three groups of animals (n=36) served as negative controls in which the rats had only uncoated rods inserted into their urethra. The subsequent two groups (n=12) served as a positive controls in which the rods inserted were coated with nanosilver and PVP. The remaining rats (n=6) did not have any biomaterials inserted.

The above-mentioned rods were placed in rats from the peritoneal cavity to the urethra. They were inserted by piercing below the exposed urinary bladder, at the site between the urinary bladder and the urethra. After drilling through a micro hole in the urethra, the rod was fixed with its rounded tip towards the external wall of the urinary bladder. The length of the rod was made so that it did not protrude from the external urethral orifice, thus enabling study of the encrustation process and to avoid the rods being pulled out or bitten by the rats. The diameter of the rod had to be twice as small as the urethra diameter, and external tip of the rod had to be rounded.

The animals remained under veterinary supervision. After 7 to 14 days after the onset of the study the rats are sacrificed. Samples of urine, blood, tissues and the rods were taken in sterile conditions. Urine and blood samples were subjected to micobiological examinations.

In serum samples, lysozyme levels and defensins activity were measured.

The surface of the rods was analysed for the colonization by microorganisms and the degree of incrustation by glycocalyx.

Isolated organisms adhering to the surface of the rods were identified and their biochemical activity characterized, including the determination of their sensitivity to antibiotics.

Prior to fixation, the tissues were analyzed for the settlement of microorganisms. Fixed samples of tissues examined morphologically and immunochemically and the presence of defensins was determined.

Characteristics of microorganisms isolated from animal tissues

It was found out that the microorganisms were identical as the strains tested (antibiogram, integrons profile, urease activity - in consent). As bacteremia/bacterinuria related with the polymer of which the rod was made, and its layer in contact with the site of activity was considered the state when the microorganism was characterized by antibiogram, integron profile, and urease activity, identical as those of the microorganism isolated from the tip or other segment of the catheter, from urine and blood of the animals in the study.

### Quantitative determinations

Bacteria isolated from blood, urine, tissues and polymer rods were counted by classical methods.

Serial dilutions of the animal fluids or homogenized tissue (urethral bioptates) were plated on the appropriate media and cultured at the temperature of 37°C for 24 hours, in aerobic and anaerobic conditions. The number of bacteria was counted and calculated for 1 ml of blood/urine or 1 g of tissue as the mean value obtained from the 3 tests performed for a bioptate taken from a single animal. The obtained segments of polymer rods were cultured and counted by means of a quantitative technique. The samples were incubated on a solid medium (5% sheep blood agar) or on another growth medium, and the colonies cultured were counted after 24 h incubation at 37°C.

### Bacterial identification system

Identification of bacteria was performed on the basis of based on biochemical activity characteristics of isolates, usually using detection systems API (bioMerieux, France), RT PCR.

### Virulence characteristics of isolates

### Quantitative analysis of activity urease of ureolytical bacteria in vitro.

Quantitative analysis of the bacteria examined was performed at various pH. The rate of urea conversion to ammonia was measured according to manufacturer recommendations (Wako Chemical). The urease activity was then expressed as µmol urea hydrolysed after 1 min, 1 mg of protein. A standard curve was obtained with NH₄Cl within the range 0.1 - 20 mg N-NH₄⁺/L.

### Antimicrobial susceptibility profile as a marker for detecting similar strains

The minimum inhibitory concentration (MIC) was determined. Bacteria were tested using the disc diffusion method on Mueller-Hinton agar, according to CSLI guidelines. In both assays, *E. coli* ATTCC 25922, *P. aeruginosa* ATTC 27853, *S. aureus* ATTC 29213, *E*. *faecalis* ATTC 29212 were used as the reference strains for antimicrobial susceptibility testing.

### Presence of integrons/transposons PCR

The phenomenon of increasing selective resistance in bacteria, resulting from ineffective treatment, is one of the biggest challenges for health care. Mobile integrons are one of the mechanisms of spreading multi-drug-resistance. Integrons are able to transfer within the bacterial genome, as well as horizontally to and from integron-positive cells. In the study, the integron pattern was determined in bacterial strains isolated from the tested samples (urine, blood, tissues, polymer rods). Resistance genes localized in the integron box were analyzed by means of PCR with specific primers.

Integron profiles are useful tools in comparing isolates which are considered as being identical.

### Effect of new biomaterials on innate immunity response

In natural physiological conditions the urinary tract is partially sterile. This phenomenon is related mainly to innate immunity response, and particularly to antimicrobial substances, such as defensins, cathelicidins, lactoferrin and lysozymes. Defensins and lysozyme are the best known and the most important for urinary tract immunity. Lysozyme levels were measured in serum, urine and tissue samples using the ELISA test, and enzymatic activity measured using the turbidimetric method. Selected defensins were measured in homogenized tissues using a sandwich ELISA test. Test RT-PCR is performed to analyze the expression of defensins in the cells of rat populations.

### Measurement of cytotoxicity of new biomaterials

It is of the utmost importance that the material from which the inserted catheters are manufactured is biocompatible, therefore, different types of surfaces and surface coatings were examined.

A number of tests for compatibility are used which allow to determine whether a structure and/or a particular coating activates the innate immune system, if the tested materials induce necrosis and/or apoptosis, if the material is cytotoxic, and if it interferes with cell proliferation. The tests are performed both *in vivo* and *in vitro.* The results of the above-mentioned tests constitute a basis for the selection of a proper material for catheter nanocoating, which material, as such, satisfies the condition of being nontoxic, inducing minimum cell death, causing no or only limited inflammation, and evoking no overt inflammation. Throughout the investigations different materials for catheter production were tested, alone or in combination with the catheter.

Several tests were applied to assess the performance of biomaterials; 1) in conformity with established (external) standards, based on the guidelines laid out in FDA Modified [ISO] Matrix (Blue Book Memorandum # G95-1, Attachement A); 2) standardized testing defined by the owner of the present invention, and 3) scientific tests aiming to extend knowledge concerning the response of the organism to the nanostructures under development.

### Catheter insertion tests:

In order to monitor the toxic effect of materials leaking from the nanocoating (urethral catheters coated in biomaterial) two rabbits are tested for each of the biomaterials used in uncoated and coated urethral catheters for 1, 4 and 12 weeks. In brief, 4 strips of nanostructure coated or uncoated material used for catheters are inserted, respectively, into the left and right paravertebral muscles using a trocar. The rabbits are monitored for toxic response, and macroscopic evaluation of the implant site carried out periodically during the experiments.

At the end of the experiment, the animals are sacrificed, macroscopic evaluation of the implant site is performed, and a photographic record is taken for subsequent evaluation. Samples of blood and muscles are collected from the site of material implantation, and frozen or fixed in 4% paraformaldehyde for histopatologic examination. Muscle tissue is processed and embedded in paraffin, and sections are prepared and stained, and tissue sections evaluated for inflammation, necrosis, fibrosis and other indicators of a toxic interaction between muscle tissue and tested material. The effectiveness of the use of extracellular metabolites of the bacteria *L. reuteri* DAN080 as a basic component of catheter's external nanocoating is justified by the following findings.

The experiments were performed on the effect of live *L. reuteri* DAN080 bacterial cultures, heat inactivated *L. reuteri* DAN080, and chitosan alpha-ketoglutarate on the immune system of laboratory animals.

Forty eight 2-month-old Sprague Dawley female rats with a weight of 140-275 g, were fed with feed adequate for the age of the animals, and watered *ad libitum.* Three days prior to experiment, all animals had catheters inserted in the jugular vein. The study was started by taking blood samples from the rats. Subsequently, the animals were administered intragastrically, by means of a gastric tube, 0.5 ml of the following preparations: suspension of the bacteria *L. reuterii* DAN080 - live and dead cells, chitosan AKG suspension, saline. Hundred twenty min. after the first blood taking the second blood sample was taken from the animals. On the second day, the rats received the same preparations once daily for 7 subsequent days. Twelve rats received live bacteria at a dose of 10⁶ cells suspended in physiological saline. The following 12 animals also received for 8 days, 10⁶ each of thermally killed cells *L. reuterii* DAN080. To the next 12 rats chitosan AKG suspension was administered, and the fourth group of animals (n=12) were administered intragastrically for 8 days, at each time 0.5 ml physiological saline. On day 8 after the final dose of the preparations administered intragastrically, blood was taken from the animals from the jugular vein. For the second time on the same day, blood was taken from all rats 120 min. after the first blood taking.

Determinations of the lysozyme activity in the blood were performed in the presence of a suspension of *Micrococcus lysodeikticus* cells of specified density, based on the absorbance value, and comparing this value with the absorbance curve plotted from a number of standard dilutions of crystal lysozyme (Sigma-Aldrich) in PBS and suspension of *M*. *lysodeikticus* cells of specified density. After 15, 30, 45, 60 min., incubation absorbance was measured at the wave length of 540 nm.

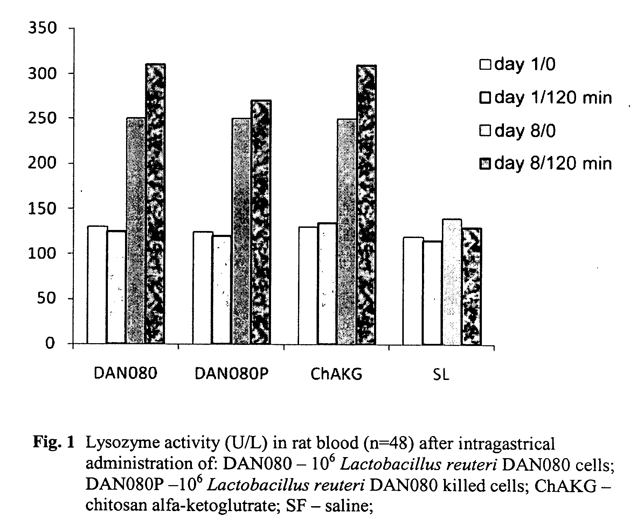

The results obtained concerning the activity of lysozyme (U/L) in the blood of rats following the intragastric administration of the tested substances, presented in Fig. 1, confirmed the stimulation of the rat immune system by live and thermally killed *L. reuteri* DAN080, and by chitosan alpha-ketoglutarate AKG.

Lysozyme is a hydrolytic enzyme released by certain phagocytes, such as macrophages and multinuclear leukocytes, plays a significant role in the control of pathogenic microorganisms. Lysozyme is also produced by Paneth cells located in the lining of the intestines. Lysozyme is especially active against Gram-positive microorganisms. Phagocytic activity of cells involves degradation, with the participation of lysozyme, of the cellular walls of bacteria, more precisely - a cleavage of glycoside bonds in peptidoglycans. An elevated lysozyme activity induced by the introduction of metabolites *of L. reuteri* DAN080 bacteria stimulated the phagocytes activation or antigen presentation to phagocytes. In this way, the function of the immune system was enhanced, mainly non-specifically. This confirms that both the live and dead cells of *L. reuteri* DAN080 show the ability to act against many pathogens. The cells as such are not recognized as dangerous by the immune system of the organism. Antimicrobial activity of the bacteria *L. reuteri* DAN080, their extracellular metabolites and chitosan alpha-ketoglutarate, is additionally enhanced, because neither live *L. reuteri* DAN080 nor their metabolites or chitosan alpha-ketoglutarate are sensitive to the activity of lysozyme, and are not hydrolyzed in contact with lysozyme.

Hyperurikemia was induced in healthy rats by blocking the activity of urate oxydase (EC 1.7.3.3) by the inhibition of purine metabolism. After a month of feeding with an addition of an inhibitor (oxonic acid, uric acid at a daily dose of 0.4 and 0.6 g, respectively), sand and stone developed in the animal's kidney (see: Bluestone R, Waisman J, Klinenberg JR. Chronic experimental hyperuricemic nephropathy. Lab Invest. 1975;33(3):273-9). This model serves for the testing the efficiency of functioning the catheter according to the invention. The catheterization of the kidney protects against the crystallization of stones.

The following facts justify including vitamin D in nanocoatings. The role of vitamin D and its active metabolites in the defense responses of the organism covers several levels. On the first level are epithelial cells which constitute a physical barrier protecting against injury and/or infection/invasion. Active hormone 1.25(OH)2 vitamin D enhances the physical barrier by stimulating genes encoding gap junction proteins, adherence genes, tight junction genes, and enhances intercellular communication (proteins: connexin 43, E-cadherin, occludin).

Vitamin D has a stimulatory effect on epithelial cells in the synthesis of antimicrobial peptides of innate immunity, including beta-defensins, cathelicidin LL-37.

Subsequently, vitamin D stimulates expression of potentially active antimicrobial peptides synthetized in macrophages/neutrophils, and increases the potential of oxygen explosion in macrophages. Besides, it enhances the neutralization of endotoxins through LL-37.

With respect to acquired immunity, vitamin D shows a suppressive effect, manifested as its capability for the inhibition of T lymphocytes proliferation. It exerts a suppressive effect on immunity dependent on the production of cytokines and immunoglobulins through activated B lymphocytes. It inhibits the activity of Th1 lymphocytes, and reduces synthesis by Th1 IF-gamma and IL-2 (stimulator of antibodies and cytokines). These lymphocytes participate in the development of disorders of the autoimmune background (e.g. type 1 diabetes, rheumatoid arthritis, autoimmune inflammation of the intestines, and multiple sclerosis).

The presence of vitamin D at the site where the mucous membrane in contact with the catheter has an ability to produce antimicrobial peptides, has a stimulatory effect on antimicrobial activity of the epithelial cells lining the lumen of the urogenital system, digestive system, genital tract, respiratory system, blood and lymphatic vessels. In the case of LPS secretion by Gram-negative bacteria (causing mainly the infections of the urogenital system), vitamin D reduces the toxic effect of endotoxins by stimulation of innate immunity effector cells, which enhances the production of antimicrobial peptides neutralizing LPS.

On the other hand, vitamin D protects against allergic reactions, which may be induced by the insertion of a catheter into a site of its use.

The experiments mentioned below confirm the positive effect of the addition of extracellular metabolites *of L. reuteri* DAN080.

Behavioral tests were performed based on an open field test for the assessment of anti-anxiety effect, for the analysis of locomotor and exploratory activity under the influence of killed and live *L. reuterii* DAN080 cells on laboratory rats.

It was possible to determine the effect of those preparations on the general profile of the behavior of the animals.

Three groups of animals were administered heat treated and live *L. reuterii* DAN080 cells for three months at a dose of 10⁶ and saline at a volume of 1 ml intragastrically, using a tube. Starting from the second month of the experiment, the dose was doubled, and divided into the administration of the preparation in the morning and in the evening. Behavioral tests were performed 3 times at monthly intervals.
1. Open field test was performed 3 times, in the first, second and the third month of the experiment. The test was performed in a plastic box of the size 100 cm x 100 cm x 40 cm (height of the wall). The square floor of the box was divided by lines into 25 equal squares. The testing was performed in conditions of a quiet and bright room. Individual behaviors of the rats were observed. Each rat in the experiment was taken out of its cage and placed in the centre of the box floor.
   a. The number of squares which the rat passed during 3 min. of observation was registered.
   b. In the same box and under the same conditions an experiment was performed on the rats consisting of a 3 min. observation of the number of withdrawals of the animal's body.
   c. In the same box and under the same conditions an experiment was conducted on the rats consisting of a 3 min. observation of the number of occurrences of the snout washing and cleaning the fur.

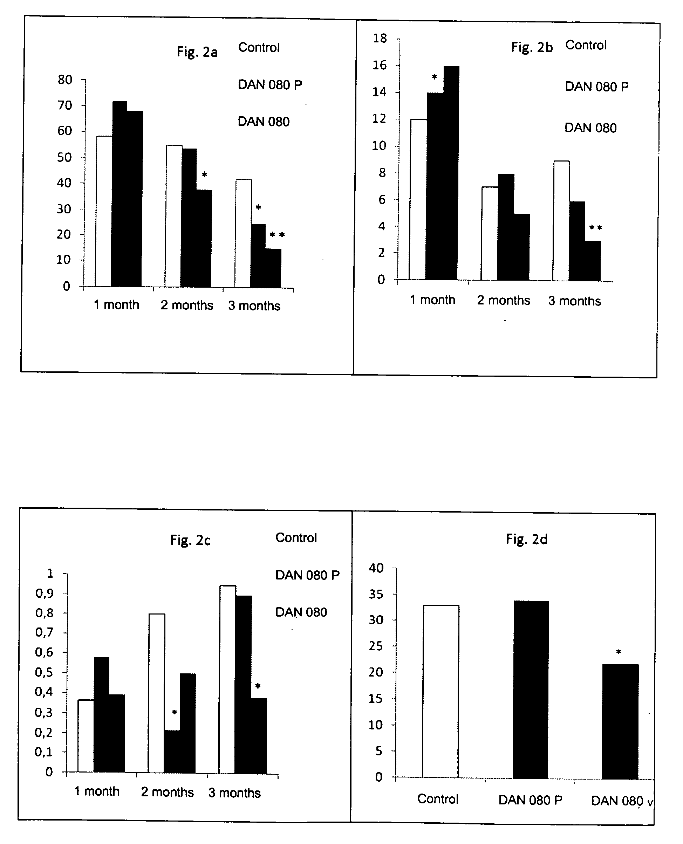

   Horizontal activity of rats was measured by the number of traversed squares. The young rats, after one month of administration of the tested preparations, showed high locomotor activity. The decrease in this horizontal activity was observed between the second and third month of the study. The least mobile, compared to the control animals, were the rats which were administered live *L. reuteri* DAN080 bacteria, followed by those receiving heat treated *L. reuteri* DAN080 (**p< 0.5, Student's t-test, *p< 0.5, *t*-test).
   During the experiment, all animals with the lapse of time and - most probably, with ageing showed a tendency towards a progressive decrease in horizontal and vertical activity.
   Vertical activity is measured by the number of occurrences of the animal body withdrawals.
   During the first month of the administration of the preparations the young rats were mobile, and those administered live *L. reuteri* DAN080 cells for at least 3 months showed a statistically significant difference in activity, compared to the control group (*p< 0.5, *t-*test). Also, compared to the control group, a statistically significant decrease in vertical activity was noted in the rats which for 2 months had received dead *L. reuteri* DAN080 cells - (*p< 0.5, *t*-test).
   In the group of animals which for 2 or 3 months received live and heat treated cells, statistically significant differences were observed (*p< 0.5, *t*-test) in the number of occurrences of the snout washing and fur cleaning, compared to the same activity performed by the control group of animals receiving exclusively saline.
   In the control group, a tendency was noted towards an increase in the number of occurrences of the snout washing and fur cleaning (observation from the 1^{st} through 3^{rd} month of saline administration).
   These data indicate that during the period between the 2^{nd} and 3^{rd} month of administration of the *L. reuteri* DAN080, the treatment exerted a calming effect on the rats. The results obtained are illustrated by Figure 2a-c, wherein the number of traversed squares (Fig. 2a), the number of rat body withdrawals (Fig. 2b), and number of occurrences of the snout washing and fur cleaning (Fig. 2c) are shown.
2. Open field test - social behavior. The experiment was carried out during the 3^{rd} month of the experiment, in the same box and under the same conditions. The only difference was that 2 rats coming from 2 different cages were placed in the box. The animals received intragastrically the same preparations, according to the above-mentioned schedule of division into groups. The behavior of each pair was observed for 7 min.
   a. In the same box and under the same conditions an experiment was conducted with a pair of rats consisting of a 7-min. observation of the number of animals' body withdrawals.
   b. In the same box and under the same conditions an experiment was carried out with a pair of rats consisting of a 7-min. observation of the number of occurrences of the snout washing and fur cleaning.
   c. In the same box and under the same conditions an experiment was conducted with a pair of rats consisting of a 7-min. observation of the number of occurrences of mutual sniffing.

The number of occurrences of body withdrawals, compared to the control group, showed a statistically significant decrease in the activity of animals receiving live *L. reuteri* DAN080 cells (*p< 0.5, *t*-test). The statistically significant decrease in the number of the snout washing and fur cleaning, and sniffing noted in the group of rats receiving live *L. reuteri* DAN080 (**p< 0.5, Student t-test) indicates both a lack of stressful effect and anxiety evoking in the animals after the administration of the test preparations.

At the same time, in all the experiments conducted, no statistically significant differences were noted in the frequency of defecations and urinations by the rats. This indicates a decrease in the rats' anxiety of their new surroundings and/or new conditions.

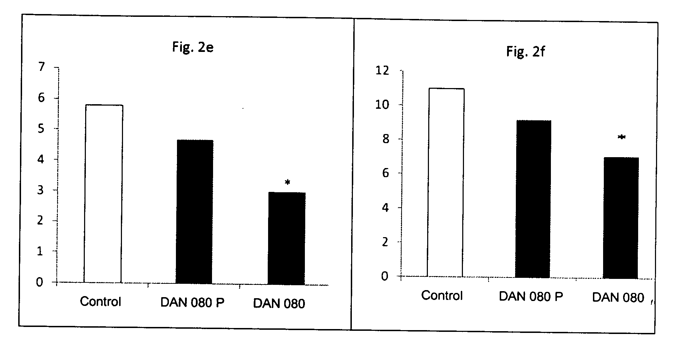

Figure 2d-2f presents the results of behavioral tests conducted on rats receiving live and heat treated *L. reuteri* DAN080 at a dose of at least 10⁶ cells/ml and physiological salt at a volume of 1 ml daily. In the open field test, the social behavior of the animals was tested by examining the number of rat body withdrawals (Fig. 2d), number of occurrences of the snout washing and fur cleaning (Fig. 2e), and number of mutual sniffings (Fig. 2f).

The experiment also confirmed a stimulatory effect of the above-tested factors in contact with epithelial cells of the mucous membranes of the body cavities other than the alimentary tract, which required catheterization.

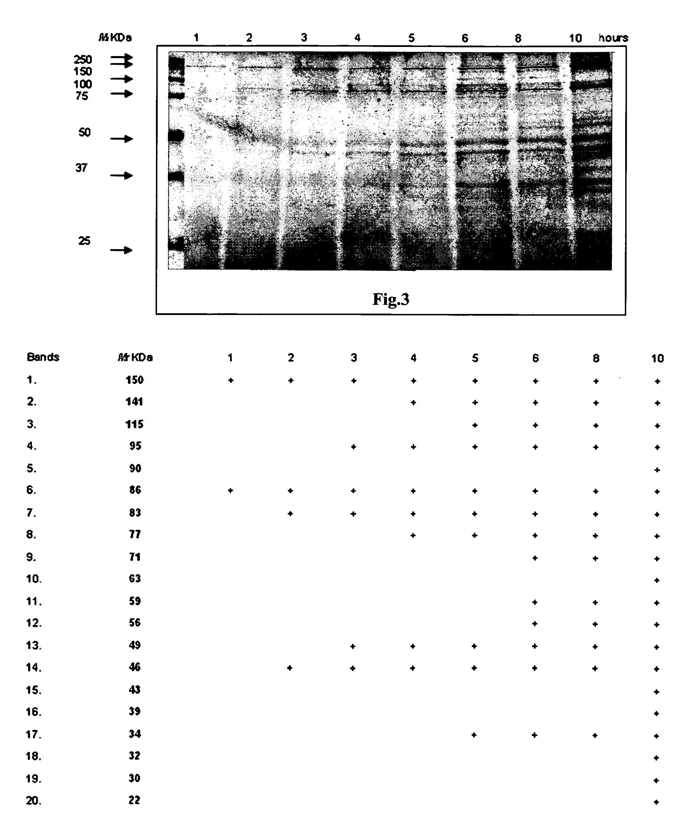

Fig. 3 illustrates the results of electrophoresis of supernatants of the *L. reuteri* DAN080 cultures.

### Example 1.

The commercially available catheters made of PVC (e.g. Galmed PL), not covered with any protective coating were processed using the methods of surface nanoengineering.

Nanocoating made of PVP was deposited after forming previously an intermediate layer on the basis of chitosan [salts], by a known method of submerging in the solution and air drying at room temperature, and optionally cross-linking by a short-term exposure to the UV radiation and/or to ultrasounds. According to the intended use of the catheter, the thickness of the intermediate layer was regulated by repeating the procedure of applying the first intermediate coating.

The intermediate coating on the basis of chitosan salts was enriched with active substances selected from a group covering thermally inactivated cultures of *L. reuteri* DAN080, chitosan alpha-ketoglutarate, small dicarboxylic acid, triclosan, silver nanoparticles, and vitamins D and E in the form of nanopowder coated with a protective coating.

Surface nanoengineering allows the manufacturing of an intermediate coating of the thickness of approximately 50,000 C-C bonds (10 nm).

The PVP polymer nanocoating of the indicated thickness, in the environment of physiological pH, is totally dissolved during usage. Gradual dissolving of the PVP layer reveals gradually the intermediate coating, wherefrom gradually active substances are released by diffusion. A single intermediate coating maintains its durability for the period up to one week, preventing the formation of biofilm, development of irritations and inflammatory states.

After 7 days of the catheter presence in the bodies of patients subjected to the urinary tract catheterization, in healthy volunteers no undesirable responses and reactions were noted.

Present studies concerning the catheters coated with hydrogel nanocoating show, for the first time, promising results with respect to the possibility of reduction of the frequency of CAUTI occurrence.

Even when a catheter is manufactured from non-invasive materials, these materials are recognized as foreign bodies by the cells of the immune system. The catheter according to the invention with currently disclosed external nanocoatings, in contact with the epithelial cells of a patient, does not exert any cytotoxic effect on the epithelium. Nanoparticles used in the study are not recognized as dangerous by the host cells. When evaluating a potential biological effect of the new material, consisting of exposing the antibacterial nanocoating to the liver tissues, the analysis showed a lack of hepatocytes reaction to the insertion of the catheter into the catchment area of the portal vein. A considerable improvement of the catheter was proposed with respect to the materials used. Due to the natural antibacterial coating, the new generation of catheters coated with hydrogel is characterized by lower contact friction, and reduces the urinary tract inflammatory processes and infections in a way similar to traditional antibiotic therapy. The catheter is cheaper and more beneficial for patients, as compared to traditional catheters.

### Example 2.

The kit according to the invention comprises a catheter as described in Example 1 above, a vial with water for injection (sterile), and a stress-reducing agent for oral administration, in the form of live or heat inactivated *L. reuteri* DAN080 cultures, at a dose of 10⁶ cells, for everyday administration for at least the period of catheterization. Depending on the patient status, the physician in charge may order administration of a stress-reducing agent also during the period preceding catheterization. The oral administration of the stress-reducing agent is recommended at 8 hours before the catheterization, or the administration directly into the body cavity at 15 min. before the catheterization.

## Claims

1. A catheter for insertion into body vessels, ducts and/or cavities, for use in prophylaxis, diagnostics and medicine, both human and veterinary, made of plastic and coated with a protective lubricant layer, **characterized in that** it has an outer nanocoatings of a biocompatible polymer capable of forming gel with water, permanently attached to said plastic either directly or through nanocoating of a polymer chemically bonded to the catheter material and having antibacterial properties, wherein at least one of the nanocoatings comprises thermally inactivated cultures of *Lactobacillus reuteri* DAN080 strain with deposit number DSM 15693, said cultures having an antimicrobial and anti-inflammatory activity, as well as an optional addition of vitamin D and E in form of nanoparticles.

2. The catheter according to claim 1, **characterized in that** the biocompatible polymer is polyvinyl pyrrolidone, and the nanocoating made of this polymer has a thickness of about 50,000 C-C bonds (10 nm).

3. The catheter according to claim 1 or 2, **characterized in that** the polymer having antibacterial properties is a salt of chitosan and small organic acid, preferably alpha-ketoglutaric acid.

4. The catheter according to anyone of the claims 1-3, **characterized in that** additional active agents selected from the group comprising chitosan alpha-ketoglutarate, chitosan citrate, chitosan lactate exhibiting antimicrobial and anti-inflammatory activity, small dicarboxylic acid, silver nanoparticles and vitamins D and E in the form of nanopowder coated with a protective coating, and the combinations thereof, are dispersed in the nanocoating made of biocompatible polymer and/or in the polymer having antibacterial properties.

5. A catheterization kit comprising the catheter according to claims 1-4 and a vial with water for injection (sterile), and an antimicrobial and anti-inflammatory agent for oral administration in the form of live or thermally inactivated cultures of *Lactobacillus reuteri* DAN080 strain with deposit number DSM 15693 at a dose of 10⁶ cells, for daily administration for the period of catheterization and/or at 8 hours prior to catheterization, or directly to the body cavity at 15 min. prior to catheterization.

6. The kit according to claim 5, **characterized in that** the water container is fixed at the tip of the catheter, and has a partition dividing water from the catheter, which partition is destroyed by the rotation of the container against the catheter when the catheter protrudes outside from the package.

## Patentansprüche

1. Katheter zur Einführung für Blutgefäße, Trakten und/oder Körperhöllen, für Prophylaxe und Diagnostik in der Humanmedizin sowie Tiermedizin, gebaut aus Kunststoff und bedeckt mit Gleitschutzhülle, **dadurch gekennzeichnet, dass** er eine äußere Nanohülle aus biokompatiblem zur Gelbildung mit Wasser fähigen Polymer hat und diese äußere Hülle mit dem genannten Kunststoff fest gebunden ist, direkt oder vermittelt durch eine Nanohülle aus einem chemisch mit Katheterstoff verbundenen und antibakterielle Eigenschaften aufweisenden Polymer, wobei wenigstens eine dieser Nanohüllen hitzeinaktivierte Kulturen von unter Nummer DSM 15693 deponiertem Stamm Lactobacillus reuteri DAN080, die antimikrobielle und antiinflammatorische Wirkung aufweisen, und auch gegebenenfalls Zusatz von Vitaminen D und E als Nanopartikel, enthält.

2. Katheter nach Anspruch 1, **dadurch gekennzeichnet, dass** das biokompatible Polymer Polyvinylpyrrolidon ist, und die Nanohülle aus diesem Polymer eine Stärke von zirka 50000 C-C Bindungen (10 nm) hat.

3. Katheter nach Anspruch 1 oder 2, **dadurch gekennzeichnet, dass** das antibakterielle Eigenschaften aufweisende Polymer eine Salz von Chitosan und der niedermolekularen organischen Säure, bevorzugt der α-Ketoglutarsäure, ist.

4. Katheter nach irgendeinem von Ansprüchen 1-3, **dadurch gekennzeichnet, dass** zusätzliche antimikrobielle und antiinflammatorische Wirkung aufweisende Wirkungstoffe, gewählt aus eine Gruppe umfassend α-Ketoglutarat von Chitosan, Chitosancitrat, Chitosanlactat, niedermolekulare Dicarbonsäure, Silbernanoteilchen und Vitamine D und E als mit Sperrschicht eingekapseltes Nanopulver, und deren Kombinationen in einer Nanohülle aus biokompatiblem Polymer und/oder in einem antibakterielle Eigenschaften aufweisenden Polymer, verteilt sind.

5. Katheter Set mit einem Katheter nach Ansprüchen 1 - 4 und ein Fläschchen mit Wasser zur Injektion (steril) und antimikrobielles und antiinflammatorisches Mittel für orale Verabreichung als lebende oder thermisch inaktivierte Kulturen von unter Nummer DSM 15693 deponiertem Stamm Lactobacillus reuteri DAN080, in einer Dosis von 10⁶ Zellen, zur täglichen Verabreichung für einen Zeitraum von Katheterisierung und/oder 8 Stunden vor Katheterisierung, oder direkt zur Korperhöhle 15 Minuten vor Katheterisierung.

6. Satz nach Anspruch 5, **dadurch gekennzeichnet, dass** der Wasserbehälter am Ende des Katheters angebracht ist und eine Wasser von dem Katheter trennende Trennwand hat, die bei der Drehung des Behälters zu dem Katheter, während Zurückziehens des Katheters aus der Verpackung, zerstört wird.

## Revendications

1. Le cathéter à introduire aux vaisseaux, conduites, et/ou cavités de corps, utile en prophylaxie, diagnostic et traitement médical ainsi que vétérinaire, fait en matière plastique et couvert d'une couche protectrice de glissement, caractéristique par le fait qu'il possède une nanocouche extérieure en polymère biocompatible capable à créer avec de l'eau le gel liée de façon fixe à la matière plastique indiquée, soit directemet, soit par l'intermédiaire de la nanocouche en polymère lié chimiquement au matériau du cathéter et possédant les propriétés antibactériennes - néanmoins, au moins l'une de ces nanocouches comprend les cultures de la souche de *Lactobacillus reuteri* DAN080 inactivées thermiquement, déposée sous le numéro DSM 15693 et lesdites cultures présentent une action antibactérienne et anti-inflammatoire, ainsi qu'elle possède éventuellement, un supplément des vitamines D et E sous forme de nanoparticules.

2. Le cathéter selon la revendication 1, caractéristique par le fait que le polymère biocompatible est polyvinylpyrrolidone et la nanocouche de ce polymère présente l'épaisseur d'env. 5000 liaisons C-C (10nm).

3. Le cathéter selon la revendication 1 ou 2, caractéristique par le fait que le polymère ayant les propriétés antibactériennes est le sel du chitosane et de l'acide de petites particules organique, préférablement de l'acide alpha-cétoglutarique.

4. Le cathéter selon n'importe quelle revendication d'entre 1-3, caractéristique par le fait que les moyens actifs supplémentaires choisis du groupe comprenant l'alpha-cétoglutarane du chitosane, le citrate du chitosane, le lactate du chitosane ayant une action antibactérienne et anti-inflammatoire, l'acide dicarboxylique de petites particules, les nanoparticules de l'argent et les vitamines D et E sous forme de nanopoudre enveloppée d'une couche protectrice ainsi que leurs combinaisons sont dispersées dans la nanocouche du polymère biocompatible et/ou dans le polymère de propriétés antibactériennes.

5. Le kit de cathétérisme, comprenant le cathéter selon les revendications 1-4 et l'ampoule avec l'eau pour injections (stérile) ainsi que le moyen antibactérien et anti-inflammatoire appliqué par voie buccale sous forme des cultures de la souche *Lactobacillus reuteri* DAN080 vives ou inactivées thermiquement, deposée sous le numéro DSM 15693, en dose de 10⁶ cellules, à administrer chaque jour dans la période de cathétérisme et/ou 8 heures avant le cathétérisme, ou directement à la cavité de corps 15 minutes avant le cathétérisme.

6. Le kit selon la revendication 5, caractéristique par le fait que la chambre avec de l'eau est montée sur l'extrémité du cathéter et possède un paroi séparant l'eau du cathéter et ledit paroi est détruit par la rotation de cette chambre envers le cathéter lors de sa sortie de l'emballage.
